# EUROPÄISCHE PATENTSCHRIFT

(11) **EP 1 519 905 B1**
(45) Veröffentlichungstag und Bekanntmachung des Hinweises auf die Patenterteilung: **02.01.2008**
(21) Anmeldenummer: 03762535.7
(22) Anmeldetag: 27.06.2003
(51) Int. Cl.: C07C 11/08, C07C 2/10

(54) **VERFAHREN ZUR OLIGOMERISIERUNG VON ALKENEN IN MEHREREN AUFEINANDER FOLGENDEN, HETEROGENEN KATALYSATORZONEN**
METHODS FOR OLIGOMERIZING ALKENES IN A NUMBER OF SUCCESSIVE, HETEROGENEOUS CATALYST ZONES
PROCEDE POUR L'OLIGOMERISATION D'ALCENES DANS PLUSIEURS ZONES CATALYTIQUES HETEROGENES SE SUCCEDANT

(30) Priorität: 03.07.2002 DE 10229763
(43) Veröffentlichungstag der Anmeldung: 06.04.2005
(73) Patentinhaber: BASF Aktiengesellschaft, 67056 Ludwigshafen (DE)
(72) Erfinder: HEIDEMANN, Thomas, 68519 Viernheim (DE); SIEBENHAAR, Ursula, 67117 Limburgerhof (DE)
(86) Internationale Anmeldenummer: PCT/EP2003/006838
(87) Internationale Veröffentlichungsnummer: WO 2004/005224

(56) Entgegenhaltungen:
- WO-A-99/25668
- DE-A- 19 922 038
- DE-A- 19 957 173

## Beschreibung

Die vorliegende Erfindung betrifft ein Verfahren zur Oligomerisierung eines Alkenstromes an einem festen, Schwefel und Nickel enthaltenden, Katalysator.

Alkene mit 2 bis 6 Kohlenstoffatomen und deren Gemische, insbesondere Alkene mit 4 Kohlenstoffatomen, stehen in großen Mengen sowohl aus FCC-Anlagen als auch aus Steamcrackern zur Verfügung. Vor allem der jeweilige C₄-Schnitt, d.h. das Gemisch aus Butenen und Butanen eignet sich nach Abtrennung des Isobutens sehr gut zur Herstellung von Oligomeren, insbesondere Octenen und Dodecenen. Sowohl die Octene als auch die Dodecene werden, im Anschluss an eine Hydroformylierung und nachfolgende Hydrierung zu den entsprechenden Alkoholen, z.B. für die Herstellung von Weichmachern oder Tensidalkoholen, verwendet.

Bei Weichmacheralkoholen wirkst sich der Verzweigungsgrad in der Regel auf die Eigenschaften des Weichmachers aus. Der Verzweigungsgrad wird durch den Iso-Index beschrieben, der die mittlere Zahl der Methylverzweigungen in der jeweiligen Fraktion angibt. So tragen z.B. n-Octene mit 0, Methylheptene mit 1 und Dimethylhexene mit 2 zum ISO-Index einer C₈-Fraktion bei. Je niedriger der Iso-Index ist, um so linearer sind die Moleküle in der jeweiligen Fraktion aufgebaut. Je höher die Linearität, d.h. je niedriger der Iso-Index ist, um so höher sind die Ausbeuten in der Oxierung und umso besser sind die Eigenschaften des damit hergestellten Weichmachers. Ein niedrigerer Iso-Index führt beispielsweise bei Phthalat-Weichmachern zu einer verminderten Flüchtigkeit und bei Weich-PVC-Sorten, welche diese Weichmacher enthalten, zu einem verbesserten Kältebruchverhalten.

Es gehört zum Stand der Technik, Alkene an Nickel- und Schwefelhaltigen heterogenen Katalysatoren zu niedermolekularen Oligomeren umzusetzen. Hierzu geeignete Katalysatoren sind weiter unten beschrieben.

Die bisher bekannten derartigen Verfahren haben jedoch den Nachteil, dass wegen des zum Reaktorausgang hin abnehmenden Gehaltes an Alken im Einsatzstoffstrom bislang in der Regel nur durch Temperaturerhöhung im Auslass-seitigen Teil des Katalysatorbettes oder Einsatz eines aktiveren Katalysators in diesem Bereich oder durch ein insgesamt vergrößertes Volumen an Katalysator ein befriedigender Umsatz der Alkene zu wenig verzweigten Alkenen möglich ist.

Der vorliegenden Erfindung lag daher die Aufgabe zugrunde, diesen Nachteilen des Standes der Technik mittels eines verbesserten Katalysatorbettes abzuhelfen.

Demgemäß wurde ein Verfahren zur Oligomerisierung eines Alkenstromes an einem festen, Schwefel und Nickel enthaltenden, Katalysator gefunden, welches dadurch gekennzeichnet ist, dass man die Oligomerisierung in zwei oder mehr als zwei aufeinander folgenden Katalysatorzonen vornimmt, wobei das molare Verhältnis von Schwefel zu Nickel in der ersten Katalysatorzone weniger als 0,5 und in der letzten Katalysatorzone 0,5 oder mehr als 0,5 beträgt und im Falle weiterer Katalysatorzonen zwischen der ersten und der letzten Katalysatorzone das molare Verhältnis von Schwefel zu Nickel dasjenige der jeweils vorangehenden Katalysatorzone, bezogen auf die Hauptrichtung des Einsatzstoffstromes, nicht unterschreitet.

Unter "Oligomeren" werden hierin Dimere, Trimere und höhere Oligomere der Alkene verstanden. Das erfindungsgemäße Verfahren eignet sich insbesondere zur Herstellung von Dimeren dieser Alkene.

Als Einsatzstoffe kommen vorzugsweise Alkene mit 2 bis 6 Kohlenstoffatomen, deren Gemische untereinander oder Gemische derartiger Alkene mit Alkanen in Betracht. Das erfindungsgemäße Oligomerisierungverfahren eignet sich besonders für die Umsetzung von Gemischen von Alkenen mit 3 und insbesondere 4 Kohlenstoffatomen, vor allem Kohlenwasserstoffströme, die 1-Buten und/oder 2-Buten und ein oder mehrere Butane enthalten und die im Wesentlichen frei sind von Isobuten.

Der Reaktor ist in der Regel ein (zylindrischer) Rohrreaktor. Alternativ kann als Reaktor eine Reaktorkaskade aus mehreren, vorzugsweise zwei oder drei, hintereinander geschalteten derartigen Rohrreaktoren (Teilreaktoren) verwendet werden, wie sie beispielsweise bekannt ist aus WO-A 99/25668 oder WO-A 01/72670.

Die Katalysatoren, an denen die Oligomerisierung durchgeführt wird, sind heterogene Katalysatoren, die Schwefel und Nickel enthalten. Sie sind allgemein bekannt, beispielsweise aus FR-A 2 641 477, EP-A 272 970, WO-A 95/14647 und WO-A 01/37989, US-A 2,794,842, US-A 3,959,400, US-A 4,511,750, US-A 5,883,036, welche hinsichtlich der darin offenbarten, Schwefel und Nickel enthaltenden Katalysatoren hiermit vollinhaltlich in Bezug genommen werden. Besonders geeignet sind hierrunter solche Katalysatoren, die zu einem geringen Verzweigungsgrad in den damit erhältlichen Oligomeren führen, vor allem jene gemäß WO-A 95/14647 oder WO-A 01/37989.

Die Gesamtheit des Katalysators, welchen der Einsatzstoff im Reaktor überstreicht, wird hierin als Katalysatorfestbett bezeichnet. Wird eine Reaktorkaskade verwendet, so ist das Katalysatorfestbett in der Regel über sämtliche Teilreaktoren der Kaskade verteilt.

Das Katalysatorfestbett in seiner Gesamtheit ist erfindungsgemäß in zwei oder mehr als zwei aufeinander folgende Katalysatorzonen unterteilt. Eine Katalysatorzone ist dabei ein Abschnitt des Katalysatorfestbettes in Strömungsrichtung des Einsatzstoffes. Eine solche Katalysatorzone zeichnet sich durch ein spezifisches Schwefel-zu-Nickel-Verhältnis gegenüber ihrer benachbarten Katalysatorzone, insbesondere - falls die Katalysatorzone zwischen zwei anderen Katalysatorzonen angeordnet ist - diesen beiden benachbarten Katalysatorzonen aus. Eine Katalysatorzone kann im Falle einer Reaktorkaskade innerhalb eines einzigen Teilreaktors angeordnet oder zusammenhängend über zwei oder mehrere aufeinander folgende Teilreaktoren verteilt sein, ohne dass das Katalysatorfestbett im ersten und letzten Teilreaktor gänzlich von dieser Katalysatorzone gebildet werden muss.

Insbesondere die Katalysatoren der ersten und der letzten Katalysatorzone können zum Zwecke der Umsetzung jeweils in einem einzelnen Reaktor oder jeweils in einer Kaskade von Reaktoren untergebracht sein.

Der Einsatzstoffstrom kann auch aufgeteilt und die so erhaltenen Teilströme dem Katalysatorfestbett an unterschiedlichen Stellen zugeführt werden, etwa bei Verwendung einer Reaktorkaskade an den Stellen welche zwischen den einzelnen Reaktoren angeordnet sind. Weiterhin ist es möglich, einen Teilstrom des gesamten Einsatzstoffstromes vor Beginn einer Katalysatorzone oder - insbesondere wenn sich eine Katalysatorzone von einem zum nächsten Teilreaktor einer Kaskade erstreckt - an der sich daraus ergebenden Teilungsstelle der Katalysatorzone zwischen den beiden Teilreaktoren zuzuführen.

Als Katalysatoren der ersten Katalysatorzone, in denen das molare Verhältnis von Schwefel zu Nickel weniger als 0,5 beträgt, werden vorzugsweise derartige Katalysatoren gemäß WO-A 95/14647 oder WO-A 01/37989 verwendet.

Als Katalysatoren für die letzte Katalysatorzone, in denen das molare Verhältnis von Schwefel zu Nickel gleich 0,5 oder mehr als 0,5 beträgt, werden vorzugsweise derartige Katalysatoren gemäß FR-A 2 641 477, EP-A 272 907, US-A 3,959,400 oder US-A 4,511,750 verwendet, insbesondere mit einem molaren Verhältnis von Schwefel zu Nickel von mehr als 0,8 und ganz besonders bevorzugt mit einem molaren Verhältnis von Schwefel zu Nickel von größer oder gleich 1.

Das erfindungsgemäße Verfahren wird vorzugsweise an einem Katalysatorfestbett durchgeführt, in dem das molare Verhältnis von Schwefel zu Nickel in der ersten Katalysatorzone weniger als 0,4 und in der letzten Katalysatorzone mehr als 0,6 beträgt.

In einer weiteren bevorzugten Auführungsform wird das erfindungsgemäße Verfahren an einem Katalysatorfestbett durchgeführt, in dem das molare Verhältnis von Schwefel zu Nickel in der ersten Katalysatorzone weniger als 0,4 und in der letzten Katalysatorzone mehr als 0,8 beträgt.

In einer weiteren bevorzugten Auführungsform wird das erfindungsgemäße Verfahren an einem Katalysatorfestbett durchgeführt, in dem das molare Verhältnis von Schwefel zu Nickel in der ersten Katalysatorzone weniger als 0,4 und in der letzten Katalysatorzone gleich oder mehr als 1 beträgt.

Die zweite und alle weiteren Katalysatorzonen des Katalysatorfestbettes werden hierin zur Unterscheidung von der ersten Katalysatorzone auch als "übrige Katalysatorzonen" bezeichnet.

Das erfindungsgemäße Verfahren wird vorzugsweise so durchgeführt, dass die Alkene des Einsatzstoffstroms in der ersten Katalysatorzone, in der das molare Verhältnis von Schwefel zu Nickel weniger als 0,5 beträgt, zu 50 bis 99, vorzugweise zu 65 bis 99, vor allem zu 80 bis 99 und insbesondere zu 90 bis 99 % umgesetzt werden.

Weiterhin wird das erfindungsgemäße Verfahren vorzugsweise so durchgeführt, dass die Alkene des Einsatzstoffstroms in den übrigen Katalysatorzonen zu 10 bis 99, vorzugweise zu 50 bis 99 % umgesetzt werden.

In einer weiteren bevorzugten Ausführungsform wird das erfindungsgemäße Verfahren so durchgeführt, dass die Alkene des Einsatzstoffstroms in der ersten Katalysatorzone zu 50 bis 99, und die nach dieser ersten Katalysatorzone unumgesetzt gebliebenen Alkene in den übrigen Katalysatorzonen zu 10 bis 99 % umgesetzt werden.

In einer weiteren bevorzugten Ausführungsform wird das erfindungsgemäße Verfahren so durchgeführt, dass die Alkene des Einsatzstoffstroms in der ersten Katalysatorzone zu 65 bis 99, und die nach dieser ersten Katalysatorzone unumgesetzt gebliebenen Alkene in den übrigen Katalysatorzonen zu 10 bis 99 % umgesetzt werden.

In einer weiteren bevorzugten Ausführungsform wird das erfindungsgemäße Verfahren so durchgeführt, dass die Alkene des Einsatzstoffstroms in der ersten Katalysatorzone zu 80 bis 95, und die nach dieser ersten Katalysatorzone unumgesetzt gebliebenen Alkene in den übrigen Katalysatorzonen zu 10 bis 99 % umgesetzt werden.

In einer weiteren bevorzugten Ausführungsform wird das erfindungsgemäße Verfahren so durchgeführt, dass die Alkene des Einsatzstoffstroms in der ersten Katalysatorzone zu 80 bis 95, und die nach dieser ersten Katalysatorzone unumgesetzt gebliebenen Alkene in den übrigen Katalysatorzonen zu 50 bis 99 % umgesetzt werden.

Weiterhin wird das erfindungsgemäße Verfahren vorzugsweise so durchgeführt, dass über alle Katalysatorzonen ein Gesamtumsatz der Alkene des Einsatzstoffstroms von mehr als 91, vorzugsweise mehr als 95 und insbesondere mehr als 97 % erreicht wird.

Die Oligomerisierungsreaktion wird im Allgemeinen bei Temperaturen von 30 bis 280, vorzugsweise von 30 bis 190 und insbesondere von 40 bis 130°C und einem Druck von im Allgemeinen 1 bis 300, vorzugsweise 5 bis 100 und insbesondere 10 bis 50 durchgeführt. Der Druck wird dabei zweckmäßigweise so gewählt, dass der Einsatzstoff bei der eingestellten Temperatur überkritisch und insbesondere flüssig vorliegt. In den einzelnen Rohrreaktoren einer Reaktorkaskade können dabei unterschiedliche Reaktionsbedingungen hinsichtlich Druck und/oder Temperatur im Rahmen dieser Druck- und Temperaturbereiche eingestellt sein.

Das erfindungsgemäße Oligomerisierungsverfahren kann adiabatisch oder isotherm durchgeführt werden.

Die Verfahrensführung ist im Übrigen dem Fachmann hinreichend bekannt, vor allem aus der WO-A 99/25668 und der WO-A 01/72670.

Nach dem Verlassen des Reaktors werden die gebildeten Oligomere in an sich bekannter Weise von den nicht umgesetzten Kohlenwasserstoffen getrennt und gewünschtenfalls in das Verfahren zurückgeführt (vgl. etwa die WO-A 95/14647). Die Auftrennung erfolgt in der Regel durch fraktionierte Destillation.

Das erfindungsgemäße Verfahren zeichnet sich gegenüber den bekannten Verfahren dieses Typs dadurch aus, dass es zu einem hohen Alken-Umsatz bei gleichzeitig niedrigem Verzweigungsgrad der so erhältlichen Oligomeren führt. Diese Wirkung konnte wegen des zum Reaktorausgang hin abnehmenden Gehaltes an Alken im Einsatzstoffstrom bislang in der Regel nur durch Temperaturerhöhung im hinteren Teil des Katalysatorbettes oder Einsatz eines aktiveren Katalysators in diesem Bereich oder durch ein insgesamt vergrößertes Volumen an Katalysator erzielt werden.

### Beispiele

### I. Katalysatoren

Das verwendete Ni(NO₃)₂-6 H₂O stammte von der Firma Fluka.

### Katalysator "1a" (S:Ni-Verhältnis = 0)

Gemäß der DE-A 43 39 713, Beispiel 1, wurde ein schwefelfreier Katalysator aus 50 Gew.-% NiO, 37 Gew.-% SiO₂ und 13 Gew.-% TiO₂ hergestellt.

### Katalysator "1b" (S:Ni-Verhältnis = 0,34)

Als Träger wurde γ-Aluminiumoxid vom Typ "D10-10" der Firma BASF AG (3 mm-Sternenstränge, BET-Oberfläche 202 m²/g, Wasseraufnahmevermögen 0,76 ml/g, Glühverlust 1,6 Gew.-%) verwendet.

200 g dieses Trägers wurden bei Raumtemperatur mit 125 ml einer Lösung aus 125 mmol 96 %-iger H₂SO₄, 361 mmol 97 %-igem Ni(NO₃)₂ · 6 H₂O und Wasser unter Rühren getränkt. Der so erhaltene Katalysator wurde 10 Stunden bei 120°C unter Luft getrocknet und 2 Stunden unter Luft bei 500°C calciniert. Danach wurde der Anteil des Nikkels ("Ni") zu 9,04 Gew.-% und der des Schwefels ("S") zu 1,67 Gew.-%, jeweils am Gesamtgewicht des erhaltenen Katalysators, sowie das molare Verhältnis von Schwefel zu Nickel ("S:Ni") im Katalysator zu 0,34 ermittelt.

Der Gehalt des fertigen Katalysators an Schwefel wurde über die quantitative Infrarot-Analyse des bei der Verbrennung des Katalysators entstandenen Schwefeldioxids ermittelt. Der Gehalt an Nikkel war über ICP-Massenspektrometrie zugänglich.

### Katalysator "1c" (S:Ni-Verhältnis = 1)

Als Träger wurde γ-Aluminiumoxid vom Typ "D10-10" der Firma BASF AG (4 mm-Stränge, BET-Oberfläche 210 m²/g, Wasseraufnahmevermögen 0,73 ml/g, Glühverlust 1,8 Gew.-%) verwendet.

400 g dieses Trägers wurden bei Raumtemperatur mit einer Lösung von 184 g NiSO₄ · 6 H₂O in Wasser unter Rühren getränkt. Das Volumen des verwendeten Wassers wurde dabei entsprechend dem Wasseraufnahmevermögen des Trägers gewählt. Der so erhaltene Katalysator wurde 16 Stunden bei 120°C unter Luft getrocknet und 2 Stunden unter Luft bei 500°C calciniert. Danach wurde der Anteil des Nikkels ("Ni") zu 7,9 Gew.-% und der des Schwefels ("S") zu 4,32 Gew.-%, jeweils am Gesamtgewicht des erhaltenen Katalysators, sowie das molare Verhältnis von Schwefel zu Nickel ("S:Ni") im Katalysator zu 1 ermittelt.

### II. Oligomerisierungen

### A) Apparatur

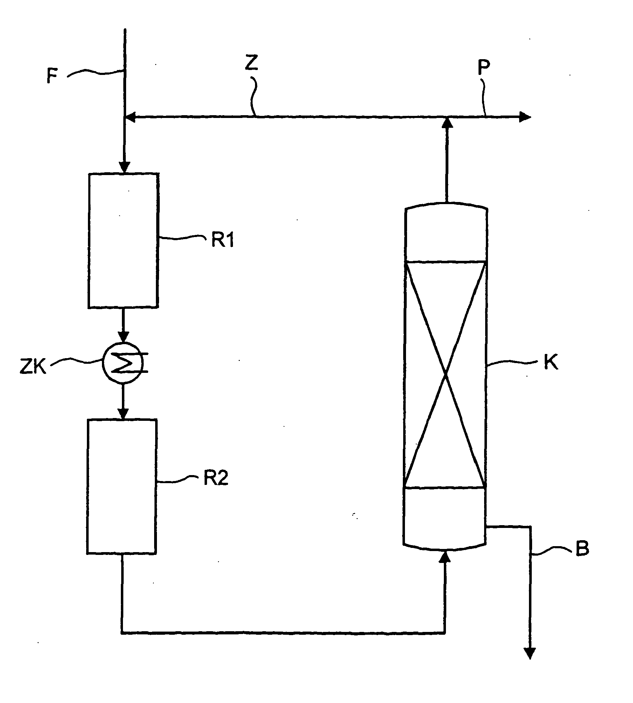

Abbildung 1 zeigt das Schema einer Vorrichtung, in der das erfindungsgemäße Verfahren kontinuierlich bei 30 bar beispielhaft durchgeführt wurde. Der Alken-haltige Strom (im Folgenden "Feed" genannt) wurde über F dem adiabatischen Teilreaktor R1 und von dort über eine Zwischenkühlung ZK dem adiabatischen Teilreaktor R2 zugeführt. Die Teilreaktoren hatten eine Länge von 4 m und einen Durchmesser von 0,08 m, somit jeweils ein Volumen von 20 Litern; wurden nur 20 Liter Katalysator eingesetzt, so wurden diese in einen der beiden Teilreaktoren verfüllt, während der zweite Steatitkugeln als Inertmaterial enthielt. Der Austrag aus Reaktor R2 wurde in der Kolonne K destillativ aufgearbeitet und das oligomere Produkt als Sumpf über B entnommen. Der Kopfstrom der Kolonne K wurde teilweise über Z in den Reaktor R1 zurückgeführt, der übrige Teil des Kopfstroms wurde über P (als "Purge"-Strom) aus der Vorrichtung geschleußt.

Der gegenüber dem Eigendruck des Raffinats II erhöhte Reaktionsdruck wurde mittels einer vorgeschalteten Reaktoreinsatzpumpe erzeugt und mittels üblicher Druckhaltevorrichtungen nach dem Reaktor reguliert.

Es kamen die Buten/Butan-Gemische gemäß Tabelle 1 zum Einsatz

**Tabelle 1 (Angaben sind Gew.-% am gesamten Einsatzstoffstrom F)**

| Gemisch | n-Butene | Butane | iso-Buten |
|---|---|---|---|
| A | 78 | 19,7 | 2,3 |
| B | 54,2 | 44,8 | 1 |
| C | 56,9 | 42,3 | 0,8 |
| D | 55,8 | 44,5 | 0,7 |
| E | 26,8 | 72,9 | 0,3 |
| F | 26,4 | 73,2 | 0,4 |

### B) Versuchsdurchführung

### B.1) Umsetzungen an einer einzigen Katalysatorzone

Die Butene/Butane-Gemische gemäß Tabelle 1 wurden bei der mittleren Temperatur T über das Volumen Vol des Katalysators Kat geleitet welcher in der Vorrichtung gemäß Abbildung 1 platziert war. Der Reaktoraustrag wurde isoliert und analysiert. Weitere Angaben zu den Versuchsparametern sowie den Versuchsergebnissen sind Tabelle 2 zu entnehmen.

### B.2)Umrechnung der Ergebnisse aus B.1 auf 2 und 3 Katalysatorzonen

Die Ergebnisse aus Abschnitt B.1 wurden rechnerisch zusammengefasst. Tabelle 3 zeigt die auf diese Weise erhaltenen Ergebnisse für die erfindungsgemäßen Abfolgen (Nr. 1, 4 und 5) von Katalysatorzonen sowie für die zu Vergleichszwecken ermittelten nicht erfindungsgemäßen Abfolgen (Nr. 2, 3, 6 und 7).

Den Versuchergebnissen ist zu entnehmen, dass bei einer Kombination von zwei oder drei Katalysatorzonen zu einem Katalysatorfestbett im Sinne der vorliegenden Erfindung - bei vergleichbaren Alken-Umsätzen und Iso-Indizes der Octene - eine um 15 bis 33 % erhöhte Raum-Zeit-Ausbeute bezüglich Octenen und Dodecenen erzielt wird, verglichen mit der Umsetzung an nicht-erfindungsgemäßen Katalysatorbetten. Zudem lassen sich diese Ergebnisse bei niedrigeren Reaktionstemperaturen am Katalysator erzielen, was erfahrungsgemäß zu einer verlängerten aktiven Lebensdauer des Katalysators (Standzeit) führt. Dadurch wird auch das Temperaturintervall vergrößert, in dem eine Umsatzsteigerung durch Temperaturerhöhung möglich ist.

**Tabelle 2**

| Gemisch | Kat | Vol [l] | T [°C] | Octene [Gew.-%] | Dodecene [Gew.-%] | C16+-Alkene [Gew.-%] | U [%] | Iso-I |
|---|---|---|---|---|---|---|---|---|
| A | 1a | 40 | 60 | 83,7 | 13,3 | 3 | 70,5 | 0,95 |
| B | 1b | 40 | 60 | 92,9 | 5,2 | 1,9 | 67,6 | 1,1 |
| C | 1b | 40 | 80 | 88,7 | 9,1 | 2,2 | 70,2 | 1,15 |
| D | 1c | 20 | 70 | 75,3 | 20,6 | 4,1 | 70,4 | 1,68 |
| E | 1a | 40 | 90 | 82,4 | 13,8 | 3,8 | 68,1 | 1,01 |
| F | 1c | 20 | 80 | 74,8 | 20,7 | 4,5 | 70 | 1,68 |

| | | | | | | | | |
|---|---|---|---|---|---|---|---|---|
| Gemisch Gemisch der Zusammensetzung gemäß Tabelle 1 Kat Verwendeter Katalysator Vol Katalysatorvolumen T Mittlere Reaktionstemperatur in der Katalysatorzone C16+-Alkene Erhaltene Alkene mit 16 oder mehr Kohlenstoffatomen U Buten-Gesamtumsatz Iso-I Iso-Index der C8-Fraktion des Oligomerisierungs-Produktes | | | | | | | | |

**Tabelle 3:**

| Nr. | Katalysatorzone 1 | | | | Katalysatorzone 2 | | | | Katalysatorzone 3 | | | | U | A | RZA | Iso-I |
|---|---|---|---|---|---|---|---|---|---|---|---|---|---|---|---|---|
| | Kat | S:Ni | Vol [l] | T [°C] | Kat | S:Ni | Vol [l] | T [°C] | Kat | S:Ni | Vol [l] | T [°C] | | | | |
| 1 (erfindungsgemäß) | 1a | 0 | 40 | 60 | 1c | 1 | 20 | 70 | - | - | - | - | 91,3 | 14,4 | 0,24 | 1,17 |
| 2 (Vergleich 1) | 1b | 0,34 | 40 | 60 | 1b | 0,34 | 40 | 80 | - | - | - | - | 90,3 | 14,1 | 0,18 | 1,12 |
| 3 (Vergleich 2) | 1a | 0 | 40 | 60 | 1b | 0,34 | 40 | 80 | - | - | - | - | 91,2 | 14,5 | 0,18 | 1,01 |
| 4 (erfindungsgemäß) | 1b | 0,34 | 40 | 60 | 1b | 0,34 | 40 | 80 | 1c | 1 | 20 | 80 | 97,1 | 15,2 | 0,15 | 1,15 |
| 5 (erfindungsgemäß) | 1a | 0 | 40 | 60 | 1b | 0,34 | 40 | 80 | 1c | 1 | 20 | 80 | 97,4 | 15,5 | 0,16 | 1,06 |
| 6 (Vergleich 3) | 1b | 0,34 | 40 | 60 | 1b | 0,34 | 40 | 80 | 1a | 0 | 40 | 90 | 96,9 | 15,2 | 0,13 | 1,11 |
| 7 (Vergleich 4) | 1a | 0 | 40 | 60 | 1b | 0,34 | 40 | 80 | 1a | 0 | 40 | 90 | 97,2 | 15,6 | 0,13 | 1,01 |

| | | | | | | | | | | | | | | | | |
|---|---|---|---|---|---|---|---|---|---|---|---|---|---|---|---|---|
| Kat Verwendeter Katalysator S:Ni Molares Verhältnis von Schwefel zu Nickel im Katalysator Vol Katalysatorvolumen T Mittlere Reaktionstemperatur in der Katalysatorzone U Buten-Gesamtumsatz A Ausbeute an Alkenen mit 8 oder 12 Kohlenstoffatomen RZA Mittlere Raum-Zeit-Ausbeute, ausgedrückt in Kilogramm Alkene mit 8 oder 12 Kohlenstoffatomen pro Liter Katalysator und Stunde Iso-I Iso-Index der C8-Fraktion des Oligomerisierungs-Produktes | | | | | | | | | | | | | | | | |

## Patentansprüche

1. Verfahren zur Oligomerisierung eines Alkenstromes an einem festen, Schwefel und Nickel enthaltenden Katalysator, **dadurch gekennzeichnet, dass** man die Oligomerisierung in zwei oder mehr als zwei aufeinander folgenden Katalysatorzonen vornimmt, wobei das molare Verhältnis von Schwefel zu Nickel in der ersten Katalysatorzone weniger als 0,5 und in der letzten Katalysatorzone 0,5 oder mehr als 0,5 beträgt und im Falle weiterer Katalysatorzonen zwischen der ersten und der letzten Katalysatorzone das molare Verhältnis von Schwefel zu Nickel dasjenige der jeweils vorangehenden Katalysatorzone, bezogen auf die Hauptrichtung des Einsatzstoffstromes, nicht unterschreitet.

2. Verfahren nach Anspruch 1, **dadurch gekennzeichnet, dass** das molare Verhältnis von Schwefel zu Nickel in der ersten Katalysatorzone weniger als 0,4 und in der letzten Katalysatorzone mehr als 0,6 beträgt.

3. Verfahren nach Anspruch 1 oder 2, **dadurch gekennzeichnet, dass** man einen Katalysator, erhältlich nach einem Verfahren bei dem man Aluminiumoxid mit einer Nickelverbindung und einer Schwefelverbindung beaufschlagt, und zwar gleichzeitig oder zuerst mit der Nickelverbindung und danach mit der Schwefelverbindung, und den so erhaltenen Katalysator anschließend trocknet und calciniert und auf diese Weise im fertigen Katalysator ein molares Verhältnis von Schwefel zu Nickel von 0,25 : 1 bis 0,38 : 1 einstellt, verwendet.

4. Verfahren nach Anspruch 1 oder 2, **dadurch gekennzeichnet, dass** man einen Katalysator, bestehend im wesentlichen aus Nickeloxid, Siliciumdioxid, Titandioxid und/oder Zirkoniumdioxid sowie gegebenenfalls Aluminiumoxid mit einem Gehalt, nach Abzug des Glühverlustes nach Temperung bei 900°C, an Nickeloxid, berechnet als NiO, von 10 bis 70 Gew.-%, 5 bis 30 Gew.-% Titandioxid und/oder Zirkoniumdioxid, 0 bis 20 Gew.-% Aluminiumoxid 20 bis 40 Gew.-% Siliciumdioxid und 0,01 bis 1 Gew.-% eines Alkalimetalloxids, mit der Maßgabe, dass sich die Anteile der einzelnen Komponenten zu 100 Gew.-% ergänzen, erhältlich durch Fällung einer Aluminium-freien oder ein gelöstes Aluminiumsalz enthaltenden Nickelsalzlösung bei einem pH-Wert von 5 bis 9 durch Zugabe dieser Nickeisalzlösung zu einer Alkaliwasserglaslösung, die festes Titandioxid und/oder Zirkoniumdioxid enthält, Trocknung und Temperung des erhaltenen Präzipitats bei 350 bis 650°C, verwendet.

5. Verfahren nach den Ansprüchen 1 bis 4, **dadurch gekennzeichnet, dass** man als Alkenstrom ein Gemisch aus Alkenrn und Alkanen mit 2 bis 6 Kohlenstoffatomen einsetzt.

6. Verfahren nach den Ansprüchen 1 bis 5, **dadurch gekennzeichnet, dass** man als Alkenstrom ein Gemisch aus Butenen und Butanen einsetzt.

7. Verfahren nach den Ansprüchen 1 bis 6, **dadurch gekennzeichnet, dass** die Alkene des Alkenstromes in der ersten Katalysatorzone zu 65 bis 99, und die nach dieser ersten Katalysatorzone unumgesetzt gebliebenen Alkene in den übrigen Katalysatorzonen zu 10 bis 99 % umsetzt.

## Claims

1. A process for oligomerizing an alkene stream over a solid catalyst comprising sulfur and nickel, wherein the oligomerization is carried out in two or more successive catalyst zones and the molar ratio of sulfur to nickel in the first catalyst zone is less than 0.5 and that in the last catalyst zone is 0.5 or more and, in the case of further catalyst zones between the first and last catalyst zones, the molar ratio of sulfur to nickel in each catalyst zone is not less than that in the preceding catalyst zone, based on the main flow direction of the feed stream.

2. The process acccording to claim 1, wherein the molar ratio of sulfur to nickel in the first catalyst zone is less than 0.4 and that in the last catalyst zone is more than 0.6.

3. The process according to claim 1 or 2, wherein a catalyst obtainable by a process in which aluminum oxide is treated with a nickel compound and a sulfur compound, either simultaneously or firstly with the nickel compound and then with the sulfur compound, and the catalyst obtained in this way is subsequently dried and calcined and a molar ratio of sulfur to nickel of from 0.25:1 to 0.38:1 is in this way set in the finished catalyst is used.

4. The process according to claim 1 or 2, wherein a catalyst which consists essentially of nickel oxide, silicon dioxide, titanium dioxide and/or zirconium dioxide and, if appropriate, aluminum oxide and has a content, after subtraction of the loss on ignition after heating at 900°C, of nickel oxide, calculated as NiO, of from 10 to 70% by weight, from 5 to 30% by weight of titanium dioxide and/or zirconium dioxide, from 0 to 20% by weight of aluminum oxide, from 20 to 40% by weight of silicon dioxide and from 0.01 to 1% by weight of an alkali metal oxide, with the proviso that the proportions of the individual components add up to 100%, and is obtainable by precipitation of an aluminum-free nickel salt solution or a nickel salt solution containing a dissolved aluminum salt at a pH of from 5 to 9 by addition of this nickel salt solution to an alkali metal water glass solution containing solid titanium dioxide and/or zirconium dioxide, drying and heating of the resulting precipitate at from 350 to 650°C is used.

5. The process according to any of claims 1 to 4, wherein the alkene stream used is a mixture of alkenes and alkanes having from 2 to 6 carbon atoms.

6. The process according to any of claims 1 to 5, wherein the alkene stream used is a mixture of butenes and butanes.

7. The process according to any of claims 1 to 6, wherein the alkenes of the alkene stream are reacted to an extent of from 65 to 99% in the first catalyst zone and the alkenes reamining unreacted after this first catalyst zone are reacted to an extent of from 10 to 99% in the remaining catalyst zones.

## Revendications

1. Procédé d'oligomérisation d'un courant d'alcènes sur un catalyseur solide contenant du soufre et du nickel, **caractérisé en ce que** l'oligomérisation est effectuée dans deux ou plus de deux zones de catalyseur successives, le rapport molaire du soufre sur le nickel dans la première zone de catalyseur étant inférieur à 0,5 et étant de 0,5 ou plus de 0,5 dans la dernière zone de catalyseur, et le rapport molaire du soufre sur le nickel, dans le cas où d'autres zones de catalyseur sont situées entre la première et la dernière zones de catalyseur, ne dépassant pas le rapport molaire de la zone de catalyseur respectivement précédente, par rapport à la direction principale du courant de substance de mise en oeuvre.

2. Procédé selon la revendication 1, **caractérisé en ce que** le rapport molaire du soufre sur le nickel dans la première zone de catalyseur est inférieur à 0,4 et est supérieur à 0,6 dans la dernière zone de catalyseur.

3. Procédé selon la revendication 1 ou 2, **caractérisé en ce qu'**un catalyseur est utilisé, que l'on peut obtenir selon un procédé dans lequel de l'oxyde d'aluminium est alimenté avec un composé de nickel et un composé de soufre, à savoir simultanément ou d'abord avec le composé de nickel et ensuite avec le composé de soufre, et dans lequel le catalyseur ainsi obtenu est finalement séché et calciné, et dans lequel un rapport molaire du soufre sur le nickel de 0,25:1 à 0,38:1 est ajusté de cette manière dans le catalyseur terminé.

4. Procédé selon la revendication 1 ou 2, **caractérisé en ce qu'**un catalyseur est utilisé, qui est constitué essentiellement d'oxyde de nickel, de dioxyde de silicium, de dioxyde de titane et/ou de dioxyde de zirconium ainsi qu'éventuellement d'oxyde d'aluminium, ayant une teneur, après évacuation de la perte par calcination après mise en température à 900°C, en oxyde de nickel, calculée en tant que NiO, de 10 à 70 % en poids, en dioxyde de titane et/ou dioxyde de zirconium de 5 à 30 % en poids, en oxyde d'aluminium de 0 à 20 % en poids, en dioxyde de silicium de 20 à 40 % en poids et en un oxyde de métal alcalin de 0,01 à 1 % en poids, sous réserve que les parts des composants individuels s'additionnent pour donner 100 % en poids, que l'on peut obtenir par précipitation d'une solution de sel de nickel exempte d'aluminium ou contenant un sel d'aluminium dissous avec une valeur de pH de 5 à 9 par ajout de cette solution de sel de nickel à une solution de verre soluble alcalin contenant du dioxyde de zirconium et/ou du dioxyde de titane solides, séchage et mise en température du précipité obtenu entre 350 et 650°C.

5. Procédé selon les revendications 1 à 4, **caractérisé en ce qu'**un mélange d'alcènes et d'alcanes ayant de 2 à 6 atomes de carbone est utilisé en tant que courant d'alcènes.

6. Procédé selon les revendications 1 à 5, **caractérisé en ce qu'**un mélange de butènes et de butanes est utilisé en tant que courant d'alcènes.

7. Procédé selon les revendications 1 à 6, **caractérisé en ce que** les alcènes du courant d'alcènes réagissent dans la première zone de catalyseur jusqu'à 65 à 99, et les alcènes demeurés non réagis après cette première zone de catalyseur réagissent dans les zones de catalyseur restantes jusqu'à 10 à 99 %.
